# EUROPEAN PATENT APPLICATION

(11) **EP 1 437 141 A1**
(43) Date of publication of application: **14.07.2004**
(21) Application number: 02763035.9
(22) Date of filing: 17.09.2002
(51) Int. Cl.: A61K 45/00, A61K 38/22, A61P 19/10

(54) **REMEDIES FOR BONE LOSS**

(30) Priority: 17.09.2001 JP 2001281108
(71) Applicant: CHUGAI SEIYAKU KABUSHIKI KAISHA, Tokyo, 115-8543 (JP)
(72) Inventor: NODA, Masaki, Shibuya-ku, Tokyo 150-0022 (JP)
(74) Representative: VOSSIUS & PARTNER
(86) International application number: PCT/JP2002/009504
(87) International publication number: WO 2003/024486

(57) **Abstract**

The object of the present invention is to provide a therapeutic agent which increases cancellous bone mass without the risk of PTH-induced loss of cortical bone in treating osteopenia.

When PTH is administered while inhibiting *in vivo* effects of osteopontin, it is possible to increase not only cancellous bone mass, but also cortical bone mass.

## Description

### TECHNICAL FIELD

The present invention relates to an agent for treating osteopenia, stimulating bone formation and increasing cortical bone mass, which comprises a substance capable of inhibiting *in vivo* effects of osteopontin, as well as at least one substance selected from the group consisting of parathyroid hormone (PTH), a PTH derivative and a PTH receptor agonist.

### BACKGROUND ART

Osteopontin (OPN), a sialic acid-rich glycoprotein with an RGD sequence, is located at a site adjacent to the clear zone of osteoclasts and is involved in cell adhesion to bone matrix through binding with integrins, particular with αvβ3 integrin. It is also present in osteoblasts. Yoshitake et al. (Proc. Natl. Acad. Sci. USA 96: 8156-8160 (1999)) reported that OPN knockout mice were resistant to ovariectomy-induced bone resorption.

Parathyroid hormone (PTH) is a linear polypeptide composed of 84 amino acids, which is secreted by parathyroid glands and acts on bone and kidney to regulate blood calcium levels. PTH acts on osteoblast-lineage cells to stimulate the production of cytokines (e.g., GM-CSF and M-CSF) and also stimulates osteoclast formation to enhance bone resorption, but PTH is also known to stimulate bone formation. For example, Reeve et al. reported that when postmenopausal patients with osteoporosis were treated with 100 µg/day PTH(1-34) given as once-daily subcutaneous injections for 6 months, the patients restored normal calcium balance and had increased cancellous bone mass. It was also reported that a higher dose (200 µg) caused stimulation of both bone formation and resorption, whereas the low dose (100 µg) allowed enhancement of bone formation while restoring normal calcium balance. They further attempted an additional study in which a total of 21 osteoporosis patients (in seven centers) were treated with PTH given as once-daily subcutaneous injections for 6 to 24 months. Although the results varied due to nonuniform background of patients, there was no improvement in calcium balance and the patients showed increased cancellous bone mass and reduced cortical bone mass (Reeve et al., Br Med J 280: 1340-1344 (1980)). Thus, PTH is known to have the effects of increasing cancellous bone mass, but reducing cortical bone mass. On the other hand, it would be important to increase bone mass of both cancellous and cortical bone to reduce fracture frequency which is critical in determining clinical outcomes in the treatment of osteopenia.

### DISCLOSURE OF THE INVENTION

The treatment of osteopenia is aimed at reducing fracture frequency. To increase bone strength and to reduce fracture frequency, it is important to increase bone mass of both cancellous and cortical bone. However, treatment with PTH involves a problem that the PTH-induced increase in cancellous bone mass is accompanied with loss of cortical bone mass.

To overcome the above problem, the inventors of the present invention have made various efforts to increase bone mass in treatment with PTH. As a result, they have found that PTH can increase not only cancellous bone mass, but also cortical bone mass when administered to an animal model in which the in vivo effects of OPN are inhibited. This finding led to the completion of the present invention.

Thus, the present invention aims to provide an ideal therapeutic agent for osteopenia, which increases not only cancellous bone mass, but also cortical bone mass.

Namely, the present invention provides a therapeutic agent for osteopenia, which comprises a substance capable of inhibiting in vivo effects of osteopontin, as well as at least one substance selected from the group consisting of parathyroid hormone (PTH), a PTH derivative and a PTH receptor agonist.

Also, the present invention provides a bone formation stimulator, which comprises a substance capable of inhibiting *in vivo* effects of osteopontin, as well as at least one substance selected from the group consisting of parathyroid hormone (PTH), a PTH derivative and a PTH receptor agonist.

Further, the present invention provides an agent for increasing cortical bone mass, which comprises a substance capable of inhibiting *in vivo* effects of osteopontin, as well as at least one substance selected from the group consisting of parathyroid hormone (PTH), a PTH derivative and a PTH receptor agonist.

### BRIEF DESCRIPTION OF DRAWINGS

Figure 1 is a graph showing changes in cancellous bone mass (BV/TV) induced by treatment with human PTH(1-34) in wild-type (WT) and OPN knockout (OPN-KO) mice.
Figure 2 is a graph showing changes in cortical bone induced by treatment with human PTH(1-34) in WT and OPN-KO mice. Figure 2A shows the thickness of cortical bone, while Figure 2B shows the area of cortical bone.
Figure 3 is a graph showing changes in cortical bone formation in the femoral mid-diaphysis induced by treatment with human PTH(1-34) in WT and OPN-KO mice. Figures 3A, 3B, 3C and 3D show periosteal bone formation rate (BFR), periosteal mineral apposition rate (MAR), endosteal BFR and endosteal MAR, respectively.
Figure 4 is a graph showing changes in cancellous bone formation in the distal end of femur induced by treatment with human PTH(1-34) in WT and OPN-KO mice. Figures 4A and 4B show BFR and MAR, respectively.
Figure 5 is a graph showing changes in the number of osteoclasts per unit trabecular perimeter of cancellous bone induced by treatment with human PTH(1-34) in WT and OPN-KO mice.
Figure 6 is a graph showing changes in urinary deoxypyridinoline (Dpyr) levels induced by treatment with human PTH(1-34) in WT and OPN-KO mice.

### BEST MODE FOR CARRYING OUT THE INVENTION

The therapeutic agent for osteopenia according to the present invention comprises a substance capable of inhibiting *in vivo* effects of osteopontin (OPN), as well as at least one substance selected from the group consisting of parathyroid hormone (PTH), a PTH derivative and a PTH receptor agonist. To enhance the PTH-induced reduction of fracture frequency, it is necessary to increase bone mass of both cancellous and cortical bone. For this purpose, the *in vivo* effects of OPN should be inhibited, as stated in the EXAMPLES section below.

As will be evident from the examples, there is no change in the number of osteoclasts or systemic bone metabolism even in the absence of OPN. On the other hand, although data are not shown here, OPN-deficient mice exhibit stimulated osteoblast differentiation when compared to wild-type mice. Namely, OPN appears to affect osteoblast-lineage cells, but not osteoclasts. OPN also appears to exert negative feedback control on the PTH-induced bone metabolic turnover.

In view of the foregoing, the therapeutic agent for osteopenia according to the present invention comprises a substance capable of inhibiting in vivo effects of OPN, in combination with at least one substance selected from the group consisting of PTH, a PTH derivative and a PTH receptor agonist.

Examples of a substance capable of inhibiting *in vivo* effects of OPN include a potential antagonist of OPN. Such a potential antagonist encompasses small organic molecules, peptides, polypeptides, antibodies and low molecular weight compounds, which inhibit or eliminate the activity or expression of OPN and/or OPN receptor integrins (preferably αvβ3 integrin) through binding to their polynucleotides and/or polypeptides.

For example, a potential antagonist encompasses small molecules which bind to and occupy binding sites on polypeptides of OPN and/or OPN receptor integrins (preferably αvβ3 integrin), thereby blocking the binding between OPN and the OPN receptor to inhibit the normal biological activity of OPN. Examples of small molecules include, but are not limited to, small organic molecules, peptides, peptide-like molecules and low molecular weight compounds. Specific examples of an antagonist capable of binding to αvβ3 integrin include an RGD peptide-like compound, SC65811 (WO97/08145).

Other potential antagonists include antisense molecules (details of which can be found in Okano, J. Neurochem. 56: 560 (1991); OLIGODEOXYNUCLEOTIDES AS ANTISENSE INHIBITORS OF GENE EXPRESSION, CRC Press, Boca Raton, FL (1988)).

Potential antagonists encompass OPN-related compounds and variants thereof.

In a case where an anti-osteopontin antibody is used as a potential OPN antagonist, antibodies that recognize the RGD sequence of OPN are preferred. There is no particular limitation on the antibodies used in the present invention, as long as they can bind to a desired antigen. It is possible to use mouse antibodies, rat antibodies, rabbit antibodies, sheep antibodies, chimeric antibodies, humanized antibodies, human antibodies and the like, as appropriate. Such antibodies may be polyclonal or monoclonal, but are preferably monoclonal because uniform antibody molecules can be produced stably. Polyclonal and monoclonal antibodies can be prepared using techniques well known to those skilled in the art. In principle, monoclonal antibody-producing hybridomas can be prepared as follows, using known techniques. Namely, a desired antigen or a desired antigen-expressing cell is used as a sensitized antigen and immunized using a standard manner for immunization. The resulting immunocytes are then fused with known parent cells using a standard manner for cell fusion, followed by screening monoclonal antibody-producing cells (hybridomas) using a standard manner for screening. Preparation of hybridomas may be accomplished according to, for example, the method of Milstein et al. (Kohler, G. and Milstein, C., Methods Enzymol. 73: 3-46 (1981)). If the antigen used is less immunogenic, such an antigen may be conjugated with an immunogenic macromolecule (e.g., albumin) before use in immunization.

In addition, expression blocking techniques may be used to inhibit the expression of a gene encoding OPN and/or OPN receptor. This blocking event may be targeted to any step of gene expression, but is preferably targeted to the transcription and/or translation steps. Exemplary known techniques of this type involve the use of antisense sequences produced *in vivo* or administered externally (see, e.g., Okano, J. Neurochem. 56: 560 (1991); OLIGODEOXYNUCLEOTIDES AS ANTISENSE INHIBITORS OF GENE EXPRESSION, CRC Press, Boca Raton, FL (1988)). Alternatively, it is possible to provide an oligonucleotide that forms a triple helix together with a target gene (see, e.g., Lee et al., Nucleic Acids Res 6: 3073 (1979); Cooney et al., Science 241: 456 (1988); Dervan et al., Science 251: 1360 (1991)). Such an oligomer may be administered as such or an important region thereof may be expressed *in vivo*.

The therapeutic agent for osteopenia according to the present invention comprises at least one substance selected from the group consisting of parathyroid hormone (PTH), a PTH derivative and a PTH receptor agonist.

The term "parathyroid hormone (PTH)" encompasses naturally occurring PTH, genetically-engineered recombinant PTH and chemically synthesized PTH. Preferred examples include human PTH composed of 84 amino acid residues (human PTH(1-84)), in particular genetically-engineered recombinant human PTH(1-84).

The term "PTH derivative" encompasses PTH fragments or metabolites, and structural analogs thereof, which can stimulate bone formation and hence increase bone mass. Also included are parathyroid hormone-related peptides as well as active fragments and analogs (WO94/01460) thereof. The activities to stimulate bone formation and to increase bone mass may be readily determined by those skilled in the art according to standard assays (Eriksen E. F. et al., Bone Histomorphometry, Raven Press, New York, 1994, pp. 1-74; Grier S. J. et al., The Use of Dual-Energy X-Ray Absorptiometry In Animals, Inv. Radiol., 1996, 31(1), pp. 50-62; Wahner H. W. and Fogelman I., The Evaluation of Osteoporosis: Dual Energy X-Ray Absorptiometry in Clinical Practice., Martin Dunitz Ltd., London, 1994, pp. 1-296). A wide variety of PTH derivatives can be found in literature and are available to those skilled in the art. Other PTH derivatives will also be apparent to those skilled in the art. Typical PTH derivatives can be found in the following documents: "Human Parathyroid Peptide Treatment of Vertebral Osteoporosis., Osteoporosis Int. 3 (Supp-1), 199-203; and "PTH 1-34 Treatment of Osteoporosis with Added Hormone Replacement Therapy: Biochemical, Kinetic and Histological Responses", Osteoporosis Int. 1, 162-170. PTH derivatives encompass PTH fragments as well as all other peptides having similar activity, including those derived by substitution of some amino acids in PTH or a fragment thereof, those derived by deletion of some amino acids in PTH or a partial peptide thereof, and those derived by addition of one or more amino acids to PTH or a partial peptide thereof. As used herein, the substitution, deletion and addition of amino acids is intended to mean amino acid substitution, deletion and addition within a range which can be achieved by well-known techniques such as site-specific mutagenesis. Preferred PTH fragments include, but are not limited to, human PTH(1-34), human PTH(1-64), human PTH(35-84) and bovine PTH(1-34). The term "PTH(1-34)" refers to a partial peptide composed of 34 amino acids between the N-terminus and amino acid 34 of PTH. More preferred PTH fragments include human PTH composed of 34 amino acid residues (human PTH(1-34)), in particular genetically-engineered recombinant human PTH(1-34). In addition, preferred examples of amino acid substitution include substitution of amino acid 8 with leucine or norleucine, substitution of amino acid 18 with leucine or norleucine, and substitution of amino acid 34 with tyrosine.

The therapeutic agent for osteopenia according to the present invention can be used to increase not only cancellous bone mass, but also cortical bone mass. It is also possible to improve bone formation rate and mineral apposition rate in cancellous bone and cortical bone (particularly in the periosteal region).

Osteopenia to be treated by the therapeutic agent of the present invention may be caused by, for example, diseases including osteoporosis.

The therapeutic agent of the present invention may be administered as a pharmaceutical composition which contains one or more pharmaceutically acceptable diluents, wetting agents, emulsifiers, dispersants, auxiliary agents, preservatives, buffers, binders, stabilizers and the like in any dosage form suitable for the intended route of administration. It may be administered parenterally or orally.

The dose of an active ingredient in the therapeutic agent of the present invention can be selected as appropriate for the physique, age and body weight of a patient, severity of the disease to be treated, elapsed time after onset of the disease, etc. For example, it is usually used at a dose of 0.01 to 1,000 mg/day/person for oral or other non-invasive administration, at a dose of 0.001 to 1,000 mg/day/person for parenteral administration by intramuscular or subcutaneous route, and at a dose of 0.0001 to 1,000 mg/day/person for parenteral administration by intravenous route. In the case of using PTH(1-34) as an active ingredient, the preferred dose for parenteral administration by intramuscular or subcutaneous route ranges from 0.01 to 100 mg/day/person, preferably 20 to 40 µg/day/person. The preferred dose for parenteral administration by intravenous route ranges from 0.001 to 100 mg/day/person, preferably 2 µg/day/person. The preferred dose for oral or other non-invasive administration ranges from 0.1 to 100 mg/day/person.

The above also applies to the bone formation stimulator and the agent for increasing cortical bone mass according to the present invention.

The kit for treating osteopenia according to the present invention comprises (a) an osteopontin inhibitor in an amount effective to inhibit *in vivo* effects of osteopontin, (b) at least one substance selected from the group consisting of parathyroid hormone (PTH), a PTH derivative and a PTH receptor agonist in an amount effective to increase bone mass, and (c) instructions for use.

### EXAMPLES

The present invention will be further described in the following example and comparative example, which are not intended to limit the scope of the invention.

Analysis procedures used in the example and comparative example are shown below.

### Determination of cancelious bone mass

Sagittal sections were prepared from the metaphyseal region of femur and tomographed using an X-ray microtomograph Musashi (Nittetsu-ELEX, Osaka, Japan). A Luzex-F automated image analysis system (Nireco, Tokyo, Japan) was then used for analysis of the image data from a 1.47 mm² square area (0.7 mm × 2.1 mm) located 0.2 mm apart from the growth plate of the distal end of femur to obtain BV/TV values.

BV/TV (bone volume/total tissue volume) denotes the unit bone mass (%), i.e., the percentage of the total trabecular volume in the total tissue volume. Namely, an increase in BV/TV means an increase in calcified bone.

### Determination of cortical bone thickness and area

Horizontal sections were prepared from the femoral mid-diaphysis and tomographed using an X-ray microtomograph Musashi (Nittetsu-ELEX, Osaka, Japan). A Luzex-F automated image analysis system (Nireco, Tokyo, Japan) was then used for analysis of the image data to determine the thickness and area of cortical bone. The thickness was evaluated at the mean value averaged over 8 measurements every 45°.

### Determination of bone formation rate (BFR) and mineral apposition rate (MAR)

In the case of cortical bone, decalcified horizontal sections (serial sections of 3 µm thickness) were prepared from the femoral mid-diaphysis and analyzed for BFR and MAR in the periosteal and endosteal regions. More specifically, a calcium chelator calcein (4 mg/kg of body weight) was subcutaneously administered 9 days and 2 days before sampling and the interval between two labeled bands was determined under a fluorescence microscope to calculate BFR and MAR.

BFR denotes bone formation rate per unit trabecular area (µm³/µm²/day).

MAR denotes the rate of increase in the distance between labeled calcified bands (µm/day).

In the case of cancellous bone, decalcified sagittal sections were prepared from the metaphyseal region and analyzed for BFR and MAR in a 1.4 mm² square area located 0.2 mm apart from the growth plate of the distal end of femur. Labeling was accomplished by subcutaneous administration of xylenol orange (100 mg/kg of body weight) 4 days before and calcein 2 days before sampling.

### Determination of TRAP-positive cells (osteoclasts)

After treatment with PTH, forefoot bone was excised from each animal. Each bone was rinsed with PBS, fixed for 7 days in 4% paraformaldehyde acid and decalcified for 3 days in 10% EDTA (pH 7.4), followed by dehydration and embedding in paraffin. Serial sections of 7 µm thickness were prepared from paraffin blocks and stained to measure TRAP (tartrate resistant acid phosphatase) activity, with Alcian blue for counter-staining. Among TRAP-positive cells attached to cancellous bone, those having at least three nuclei were counted using 15 serial sections of 7 µm thickness prepared from each bone. Each of the 15 sections was taken at five-section intervals from a total of 75 sections per bone. The number of multinucleated TRAP-positive cells in all 15 sections was summed to give the total number of osteoclasts per unit trabecular perimeter.

### Analysis of urinary deoxypyridinoline

At 26 days after treatment with PTH, a 24-hour urine sample was collected from each mouse (10 samples per group). As a marker of bone metabolism, the level of urinary deoxypyridinoline (Dpyr) was measured for each sample using an ELISA kit (Metra Biosystems, San Diego, CA) in accordance with the manufacturer's protocol.

### Statistical analysis

All data were expressed as mean ± standard error, and the statistical significance was assessed by Fisher's test.

### Example 1

To create an animal model in which the *in vivo* effects of OPN were inhibited, strain 129 female mice at 7 weeks of age were modified into knockout mice deficient in the OPN gene (OPN-KO mice), as described in Rittling et al., J. Bone Miner. Res., 13: 1101-1111, 1998. These mice were singly used for PTH treatment test. They were divided into groups of 6 mice each.

Recombinant human PTH(1-34) (Bachem, Torrance, CA) was dissolved in acidified physiological saline supplemented with 0.1% bovine serum albumin (Sigma Chemical Co.-Aldrich, St. Louis, MO). The OPN-KO mice were subcutaneously administered with 80 µg/kg of body weight human PTH(1-34) for 4 weeks on a five-days-a-week basis. Mice in the control group were administered with physiological saline alone.

The effect of PTH on bone metabolism in OPN-deficient mice was tested as follows.

### (1) Cancellous bone mass

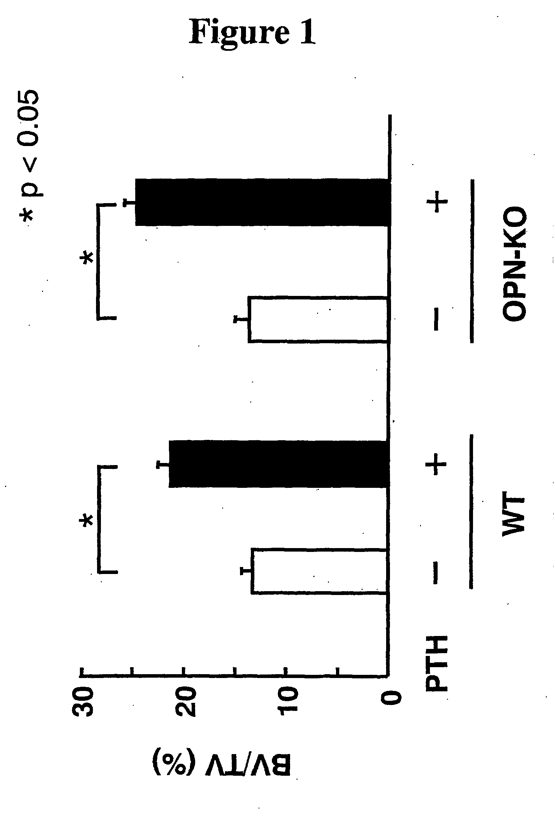

Figure 1 is a graph showing changes in cancellous bone mass (%) induced by treatment with human PTH(1-34) in WT and OPN-KO mice. As shown in Figure 1, in the case of OPN-KO mice, BV/TV was 13.45 ± 2.88% in the group receiving physiological saline (PTH(-) group), whereas BV/TV was 24.41 ± 2.85% in the group receiving PTH (PTH(+) group). Treatment with PTH caused a statistically significant increase (p<0.05) in cancellous bone mass of OPN-KO mice.

### (2) Thickness and area of cortical bone

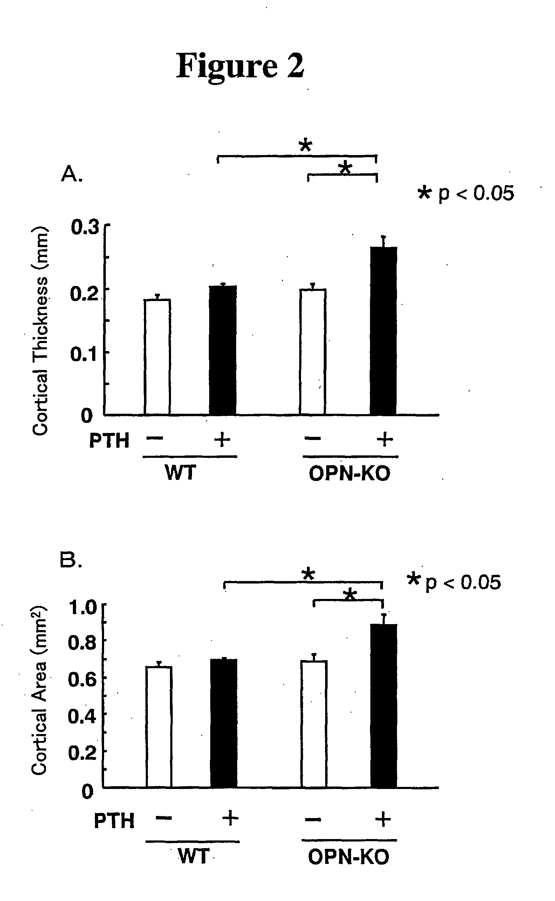

Figure 2A is a graph showing changes in the thickness (mm) of cortical bone induced by treatment with human PTH(1-34) in WT and OPN-KO mice. In the case of OPN-KO mice, the thickness of cortical bone was 0.20 ± 0.02 mm in the PTH(-) group, whereas the thickness was increased to 0.26 ± 0.04 mm in the PTH(+) group. The difference found was statistically significant (p<0.05).

Figure 2B is a graph showing changes in the area (mm²) of cortical bone induced by treatment with human PTH(1-34) in WT and OPN-KO mice. In the case of OPN-KO mice, the area of cortical bone was 0.69 ± 0.09 mm² in the PTH(-) group, whereas the area was 0.88 ± 0.13 mm² in the PTH(+) group. Treatment with PTH caused a statistically significant increase (p<0.05) in the area of cortical bone.

### (3) BFR and MAR in cortical bone

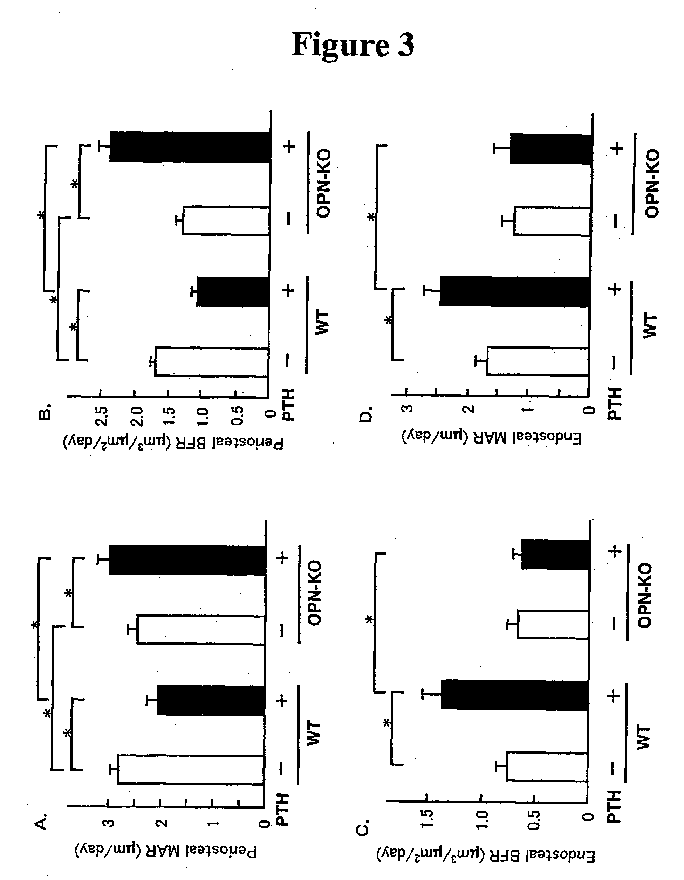

To examine bone formation mechanisms, each animal was measured for its BFR and MAR in the periosteal and endosteal regions of cortical bone in the femoral mid-diaphysis. Figure 3 is a graph showing changes in cortical bone formation in the femoral mid-diaphysis induced by treatment with human PTH(1-34) in WT and OPN-KO mice.

Periosteal BFR (Figure 3A) and MAR (Figure 3B) were 1.29 ± 0.10 µm³/µm²/day and 2.44 ± 0.18 µm/day, respectively, in the PTH(-) group, whereas they were 2.38 ± 0.18 µm³/µm²/day and 2.97 ± 0.25 µm/day, respectively, in the PTH(+) group. Treatment with PTH caused about a 1.8-fold increase in BFR and about a 1.2-fold increase in MAR, each of which was statistically significant (p<0.05).

In contrast, endosteal BFR (Figure 3C) and MAR (Figure 3D) were 0.66 ± 0.10 µm³/µm²/day and 1.25 ± 0.20 µm/day, respectively, in the PTH(-) group, whereas they were 0.62 ± 0.09 µm³/µm²/day and 1.33 ± 0.28 µm/day, respectively, in the PTH(+) group. Treatment with PTH had little effect on these parameters.

### (4) BFR and MAR in cancellous bone

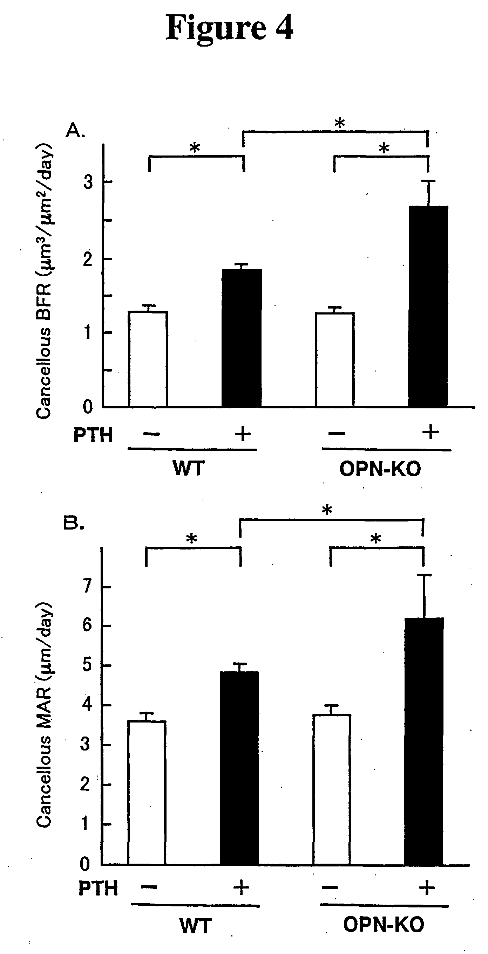

BFR and MAR were also measured for cancellous bone. Figure 4 is a graph showing changes in cancellous bone formation in the distal end of femur induced by treatment with human PTH(1-34) in WT and OPN-KO mice.

BFR (Figure 4A) and MAR (Figure 4B) were 1.27 ± 0.08 µm³/µm²/day and 3.75 ± 0.23 µm/day, respectively, in the PTH(-) group, whereas they were 2.68 ± 0.34 µm³/µm²/day and 6.21 ± 1.11 µm/day, respectively, in the PTH(+) group. Treatment with PTH caused about a 2.1-fold increase in BFR and about a 1.7-fold increase in MAR, each of which was statistically significant (p<0.05).

### (5) Number of osteoclasts

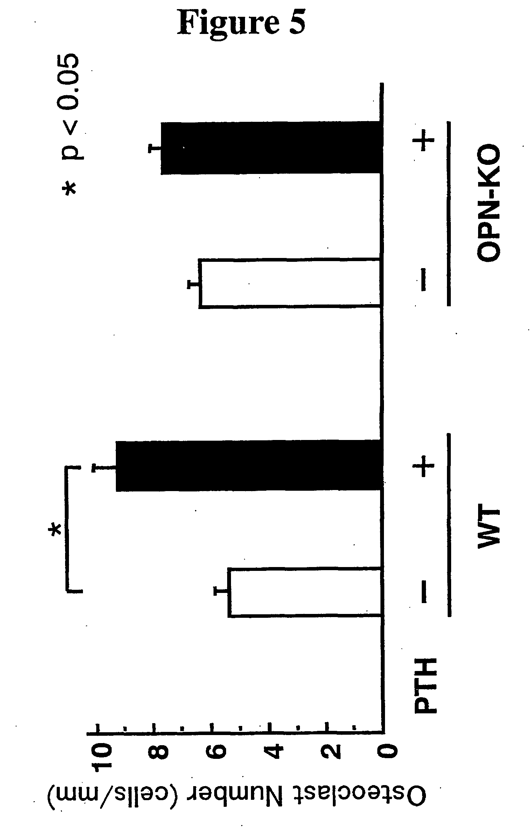

Figure 5 is a graph showing changes in the number of osteoclasts per unit trabecular perimeter induced by treatment with human PTH(1-34) in WT and OPN-KO mice. In the case of OPN-KO mice, the number of osteoclasts per unit area of cancellous bone trabeculae was 6.3 ± 0.9 cells/mm in the PTH(-) group, whereas it was increased to 7.6 ± 1.0 cells/mm in the PTH(+) group.

### (6) Urinary excretion of Dpyr

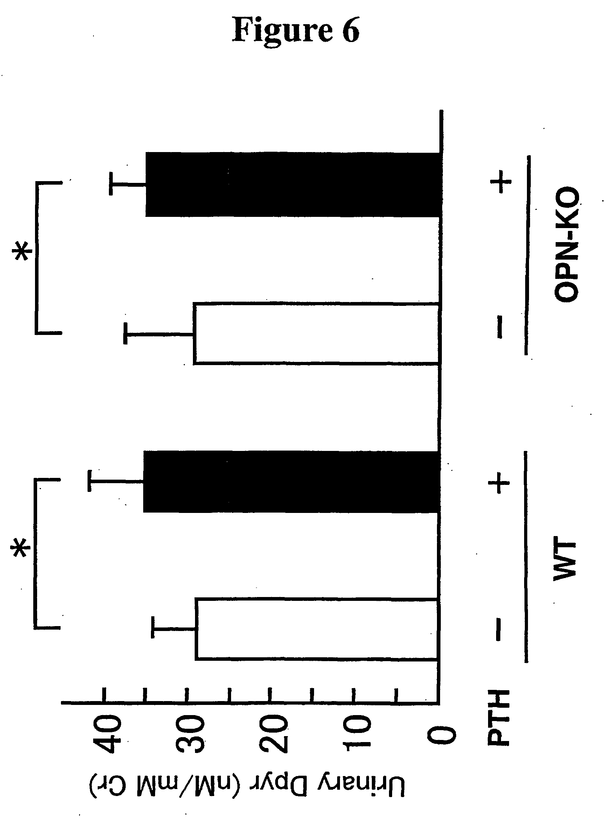

To evaluate changes in systemic bone resorption induced by treatment with PTH, each mouse was assayed for its urinary Dpyr level. Figure 6 is a graph showing changes in urinary Dpyr levels induced by treatment with human PTH(1-34) in WT and OPN-KO mice. The urinary Dpyr level was 28.93 ± 8.54 nM/mM Cr in the PTH(-) group, whereas it was 34.67 ± 4.52 nM/mM Cr in the PTH(+) group. Treatment with PTH caused about a 1.2-fold increase in systemic bone resorption, which was statistically significant (p<0.05).

### Comparative Example 1

The effect of PTH on bone metabolism was also examined in the same manner as shown in Example 1, using wild-type strain 129 female mice (7 weeks of age). These mice were divided into groups of 6 mice each.

### (1) Cancellous bone mass

As shown in Figure 1, BV/TV was 13.14 ± 2.27% in the PTH(-) group, whereas BV/TV was 21.17 ± 2.68% in the PTH(+) group.

As already reported by Jilka et al. (J. Clin. Invest. vol. 104: pp. 439-446, 1999), 4-week treatment with PTH caused a statistically significant increase (p<0.05) in cancellous bone mass of WT mice. However, there is no great difference in the magnitude of increase between OPN-KO and WT mice.

### (2) Thickness and area of cortical bone

The thickness of cortical bone was 0.18 ± 0.02 mm in the PTH(-) group and 0.20 ± 0.01 mm in the PTH(+) group (Figure 2A). Treatment with PTH caused a slight increase in the thickness of cortical bone, but there was no great change in this parameter.

As shown in Figure 2A, there was no great difference in the thickness of cortical bone between the PTH(-) groups of WT and OPN-KO mice. In contrast, the difference found in the PTH(+) groups was statistically significant (p<0.05) when compared between WT and OPN-KO mice.

The area of cortical bone was 0.65 ± 0.07 mm² in the PTH(-) group and 0.69 ± 0.03 mm² in the PTH(+) group (Figure 2B). Treatment with PTH caused a slight increase in the area of cortical bone, but there was no great change in this parameter.

As is evident from Figure 2B, there was no great difference in the area of cortical bone between the PTH(-) groups of WT and OPN-KO mice. In contrast, treatment with PTH caused a statistically significant increase (p<0.05) in the area of cortical bone in OPN-KO mice although it caused only a slight increase in WT mice. When compared between the PTH(+) groups of WT and OPN-KO mice, the difference found in the area of cortical bone was statistically significant (p<0.05).

### (3) BFR and MAR in cortical bone

Periosteal BFR (Figure 3A) and MAR (Figure 3B) were 1.67 ± 0.08 µm³/µm²/day and 2.79 ± 0.15 µm/day, respectively, in the PTH(-) group, whereas they were 1.07 ± 0.08 µm³/µm²/day and 2.05 ± 0.21 µm/day, respectively, in the PTH(+) group. Treatment with PTH caused about a 0.6-fold increase in periosteal BFR and about a 0.7-fold increase in periosteal MAR, indicating significant inhibition of bone formation (p<0.05).

Endosteal BFR (Figure 3C) and MAR (Figure 3D) were 0.76 ± 0.11 µm³/µm²/day and 1.68 ± 0.18 µm/day, respectively, in the PTH(-) group, whereas they were 1.37 ± 0.18 µm³/µm²/day and 2.44 ± 0.29 µm/day, respectively, in the PTH(+) group. Contrary to periosteal BFR and MAR, treatment with PTH caused statistically significant increases (p<0.05) in endosteal BFR and MAR (about 1.8-fold and 1.5-fold increases, respectively).

This indicated that the lack of OPN blocked the inhibitory effect of PTH on cortical bone formation and, in turn, allowed PTH-stimulated cortical bone formation, particularly by the action through the periosteal region.

### (4) BFR and MAR in cancellous bone

In cancellous bone, BFR (Figure 4A) and MAR (Figure 4B) were 1.29 ± 0.08 µm³/µm²/day and 3.61 ± 0.19 µm/day, respectively, in the PTH(-) group, whereas they were 1.85 ± 0.08 µm³/µm²day and 4.84 ± 0.21 µm/day, respectively, in the PTH(+) group. Treatment with PTH caused statistically significant increases (p<0.05) in BFR and MAR in the metaphyseal cancellous bone region (about 1.4-fold and 1.3-fold increases, respectively).

There was no difference in BFR and MAR between the PTH(-) groups of WT and OPN-KO mice. However, the lack of OPN caused statistically significant elevations (p<0.05) in the magnitude of PTH-induced increases in MAR and BFR (about 1.4-fold and 1.3-fold elevations, respectively).

### (5) Number of osteoclasts

The number of osteoclasts per unit trabecular perimeter of cancellous bone trabeculae was 5.4 ± 1.1 cells/mm in the PTH(-) group, whereas it was increased to 9.2 ± 1.8 cells/mm in the PTH(+) group. The difference found was statistically significant (p<0.05)(Figure 5).

As shown in Figure 5, there was no statistically significant difference in the number of osteoclasts between WT and OPN-KO mice, suggesting that bone resorption occurred at the same level between WT and OPN-KO mice.

### (6) Urinary excretion of Dpyr

The urinary Dpyr level was 28.72 ± 5.36 nM/mM Cr in the PTH(-) group, whereas it was 34.86 ± 6.90 nM/mM Cr in the PTH(+) group. Treatment with PTH caused about a 1.2-fold increase in systemic bone resorption, which was statistically significant (p<0.05)(Figure 6).

There was no difference in PTH-induced changes in urinary Dpyr levels between WT and OPN-KO mice (Figure 6). This would support the finding stated above that there is no PTH-induced change in the number of osteoclasts per unit area even in the absence of OPN.

### INDUSTRIAL APPLICABILITY

It has been shown that treatment with PTH in the absence of OPN effects causes not only an increase in cancellous bone mass, but also statistically significant increases in the thickness and area of cortical bone, thus enhancing the activation of PTH-induced bone formation. The therapeutic agent of the present invention is therefore useful in treating osteopenia.

## Claims

1. A therapeutic agent for osteopenia, which comprises a substance capable of inhibiting *in vivo* effects of osteopontin, as well as at least one substance selected from the group consisting of parathyroid hormone (PTH), a PTH derivative and a PTH receptor agonist.

2. The therapeutic agent for osteopenia according to claim 1, wherein the substance capable of inhibiting *in vivo* effects of osteopontin is a substance that prevents the expression of the osteopontin gene.

3. The therapeutic agent for osteopenia according to claim 1, wherein the substance capable of inhibiting *in vivo* effects of osteopontin is a substance that blocks the binding between osteopontin and a receptor thereof.

4. The therapeutic agent for osteopenia according to claim 3, wherein the receptor of osteopontin is αvβ3 integrin.

5. The therapeutic agent for osteopenia according to any one of claims 1 to 4, wherein osteopenia is caused by osteoporosis.

6. A bone formation stimulator, which comprises a substance capable of inhibiting *in vivo* effects of osteopontin, as well as at least one substance selected from the group consisting of parathyroid hormone (PTH), a PTH derivative and a PTH receptor agonist.

7. An agent for increasing cortical bone mass, which comprises a substance capable of inhibiting *in vivo* effects of osteopontin, as well as at least one substance selected from the group consisting of parathyroid hormone (PTH), a PTH derivative and a PTH receptor agonist.

8. A kit for treating osteopenia, which comprises (a) an osteopontin inhibitor in an amount effective to inhibit *in vivo* effects of osteopontin, (b) at least one substance selected from the group consisting of parathyroid hormone (PTH), a PTH derivative and a PTH receptor agonist in an amount effective to increase bone mass, and (c) instructions for use.

9. A kit for stimulating bone formation, which comprises (a) an osteopontin inhibitor in an amount effective to inhibit *in vivo* effects of osteopontin, (b) at least one substance selected from the group consisting of parathyroid hormone (PTH), a PTH derivative and a PTH receptor agonist in an amount effective to increase bone mass, and (c) instructions for use.

10. A kit for increasing cortical bone mass, which comprises (a) an osteopontin inhibitor in an amount effective to inhibit *in vivo* effects of osteopontin, (b) at least one substance selected from the group consisting of parathyroid hormone (PTH), a PTH derivative and a PTH receptor agonist in an amount effective to increase bone mass, and (c) instructions for use.

11. A method for treating osteopenia, which comprises administering to a patient in need thereof, an osteopontin inhibitor in an amount effective to inhibit *in vivo* effects of osteopontin in combination with at least one substance selected from the group consisting of parathyroid hormone (PTH), a PTH derivative and a PTH receptor agonist in an amount effective to increase bone mass.

12. A method for stimulating bone formation, which comprises administering to a patient in need thereof, an osteopontin inhibitor in an amount effective to inhibit *in vivo* effects of osteopontin in combination with at least one substance selected from the group consisting of parathyroid hormone (PTH), a PTH derivative and a PTH receptor agonist in an amount effective to increase bone mass.

13. A method for increasing cortical bone mass, which comprises administering to a patient in need thereof, an osteopontin inhibitor in an amount effective to inhibit *in vivo* effects of osteopontin in combination with at least one substance selected from the group consisting of parathyroid hormone (PTH), a PTH derivative and a PTH receptor agonist in an amount effective to increase bone mass.

14. Use of a substance capable of inhibiting *in vivo* effects of osteopontin together with at least one substance selected from the group consisting of parathyroid hormone (PTH), a PTH derivative and a PTH receptor agonist, for the manufacture of a therapeutic agent for osteopenia.
